Europäisches Patentamt

European Patent Office

Office européen des brevets

(11) Publication number: **0 534 995 B1**

# EUROPEAN PATENT SPECIFICATION

(45) Date of publication of patent specification: **28.12.94**  (51) Int. Cl.⁵: **A61K  47/48**, A61K 31/63

(21) Application number: **91909437.5**

(22) Date of filing: **20.05.91**

(86) International application number:
**PCT/IT91/00043**

(87) International publication number:
**WO 91/17774 (28.11.91 91/27)**

(54) INCLUSION COMPOUNDS OF NIMESULIDE WITH CYCLODEXTRINS.

(30) Priority: **22.05.90 IT 2039390**

(43) Date of publication of application:
**07.04.93 Bulletin  93/14**

(45) Publication of the grant of the patent:
**28.12.94 Bulletin  94/52**

(84) Designated Contracting States:
**AT BE CH DE DK ES FR GB GR IT LI LU NL SE**

(56) References cited:
**FR-A- 2 190 460**

**Nambu et al. Chem. Pharm. Bull. 1978 vol 26, 3609-3612**

**Chem. Pharm. Bull., vol. 23, no. 12,1975, pages 3026-3068, M. Kurozumi et al.:"Inclusion compounds of non-steroidal antiflammatory and other slightly water soluble drugs with alpha- and beta-cyclodextrins in powered form"**

(73) Proprietor: **BOEHRINGER INGELHEIM ITALIA S.p.A.**
**Via Pellicceria, 10**
**I-50123 Firenze (IT)**

(72) Inventor: **BIETTI, Giuseppe**
**Via Lario, 21**
**I-Milano (IT)**
Inventor: **DUBINI, Enrica**
**Via Muratori, 54**
**I-Milano (IT)**
Inventor: **MAFFIONE, Grazia**
**Via Panizzi, 6/A**
**I-Milano (IT)**
Inventor: **VIDI, Aldo**
**Via Donizetti, 4**
**I-27029 Vigevano (IT)**

(74) Representative: **Aimi, Luciano et al**
**c/o Società Italiana Brevetti S.p.A.**
**Via Carducci 8**
**I-20123 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Chem. Pharm. Bull., vol. 26, no. 4, April 1978, pages 1162-1167, Phar. Society of Japan, K. Ukeama et al.:"Inclusion complexation..".

**Description**

The present invention relates to new compounds obtained by complexation of nimesulide with cyclodextrins, to the processes for their preparation and to pharmaceutical compositions containing them.

Nimesulide is the International Non-proprietary name for 4-nitro-2-phenoxy-methansulfonanilide, patented in USA (US Pat. 3,480,597). Nimesulide is a non-steroidal anti-inflammatory drug useful in the treatment of inflammatory and pain states, such as chronic rheumatoid arthritis or osteoarthritis, otorhinolaryngological diseases, soft tissues, oral cavity inflammation and postoperative pain states. Nimesulide presents the usual side-effects associated with non-steroidal antiinflammatory drugs. Furthermore it is known that nimesulide has a very poor water solubility, and this gives rise to well-known difficulties, e.g. preparation of suitable pharmaceutical compositions, poor rate of release and therefore a poor and erratic bioavailability.

It has been now found, and this is the object of the present invention, that nimesulide can be advantageously complexed by inclusion into an unsubstituted or substituted $\alpha$-, $\beta$- or $\gamma$-cyclodextrin or a hydrate thereof.

The inclusion compound so obtained is useful as analgesic and antiinflammatory drug and possesses good water solubility, it is absorbed in a more efficent and rapid way, and it shows a virtually improved gastric tolerability.

The efficacy of the new inclusion compound has been tested by known pharmacological methods.

Cyclodextrins are cyclic oligosaccharides consisting of at least six glucopyranose units which are joined together by $\alpha$-1,4-glucosidic bonds. Although, cyclodextrins with up to 12 glucose residues are known, only three homologs have been studied extensively: $\alpha$-, $\beta$-, and $\gamma$-cyclodextrins consisting of 6,7 and 8 glucose residues respectively. The cyclodextrin molecules are of cylindrical shape, having a central axial cavity. The outer surface of these molecules is hydrophilic, while the internal cavity is of apolar character. This feature allows other molecules ("guest molecules") or part of them, which are less polar than water and are of suitable dimensions, to penetrate in the lipophilic cavity of the inner part of the cylindric cyclodextrin molecule, forming thereby the inclusion compound.

Suitable cyclodextrins which may be used according to the present invention are unsubstituted or substituted $\alpha$-, $\beta$- or $\gamma$-cyclodextrins or hydrates thereof. Examples of substitued cyclodextrins include surphur-containing cyclodextrins, nitrogen-containing cyclodextrins, methylated cyclodextrins, hydroxypropyl-$\beta$-cyclodextrins.

Conveniently a single unsubstituted cyclodextrin is employed according to the present invention, particularly preferred is $\beta$-cyclodextrin,

For the preparation of the inclusion compounds of nimesulide with a cyclodextrin in a molar ratio comprised between 1 : 0.5 and 1 : 15, preferably 1 : 1 the following known methods are available:

    a) the solutions of cyclodextrin in water and of nimesulide in a suitable organic solvent are strirred vigorously and the obtained complex is separated by filtration;

    b) nimesulide and cyclodextrin are dissolved under stirring in a water-ammonia solution at a temperature from 0° to 100° C, preferably at room temperature. The desired compound is then obtained by freeze-drying or atomization;

    c) a mixture of nimesulide and cyclodextrin in water is stirred for several days and the complex is obtained by evaporation under reduced pressure.

The following examples illustrate better the present invention, but they are not to be in any way considered limitative of the scope of the invention itself:

Example 1

A solution of nimesulide (0.5 g. 1.62 mmoles) in methylene chloride (15 ml) was added to a solution of $\beta$-cyclodextrin (1.84 g, 1.62 mmoles) in water (250 ml). After one night shaking at room temperature, the precipitate was collected by filtration and dried under vacuo at 40° C.

Example 2

A mixture of nimesulide (0.31 g, 1 mmoles) and $\beta$-cyclodextrin (1.13 g, 1 mmoles) in water (160 ml) was stirred at 60° C for 8 days. The solution then cooled at room temperature, evaporated to dryness and the residue was dried in vacuo at 40° C.

Example 3

Nimesulide (3 g, 9.73 mmoles) and β-cyclodextrin (11.05 g, 9.73 mmoles) were added under stirring in water (650 ml containing 2.5 ml of aqueous 30% ammonium hydroxide). The mixture was stirred one night at room temperature and the resulting solution was freeze-dried. The residue was treated with ethyl acetate, filtered and dried in vacuo at 40° C.

Example 4

Nimesulide (31 g, 0.1 moles) and β-cyclodextrin (283 g, 0.25 moles) were suspended in water (6500 ml) and a solution of aqueous 30 % ammonium hydroxide (30 ml) was added at room temperature. After one night the solution was dried by using a Uniglatt laboratory spray-dryer to give a granulate corresponding to the title complex.

The inclusion compound so obtained was characterized as follows:

1. Quantitative determination of nimesulide complexed by β-cyclodextrin.

The quantity of nimesulide included in the complex with β-cyclodextrin is calculated by High Pressure Liquid Chromatography (HPLC) whose sperimental conditions are reported as follows:

| | |
|---|---|
| - Mobile phase | phosphate buffer 0.002 M pH 6.88: acetonitrile in the ratio 70 : 30 |
| - Flow rate | 1 ml/min. |
| - Temperature | 40° C |
| - Column | spheric RPs 5 μm (Hewlett-Packard) |
| - λ detection | 400 nm |
| - nimesulide ritention time | 4 min ca. |

2. Differential Scanning Calorimetry (D.S.C.)

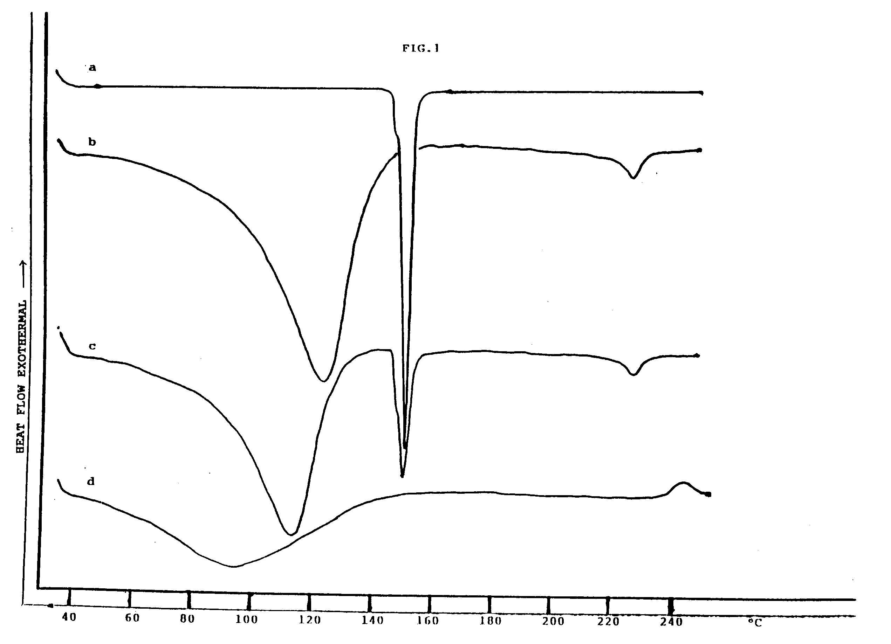

Figure 1 shows the thermograms concerning nimesulide (trace a), β-cyclodextrin (trace b), physical mixture of both (trace c) and inclusion compound (trace d).

Nimesulide melts at 149° C and does not show any decomposition up to 240° C. β-cyclodextrin shows an endothermic event between 60° to 140°C correspondig to the elimination of water.

The trace of the physical mixture shows the thermal characteristics of both nimesulide and β-cyclodextrin.

The trace of the inclusion compound nimesulide-β -cyclodextrin, after the initial loss of weight at a temperature below 140° C attributable to the absorbed moisture, does not show any other thermic event up to 240° C. Therefore, the disappearance of the melting peak at 149° C of nimesulide shows that the inclusion compound has take place.

3. Infrared spectroscopy

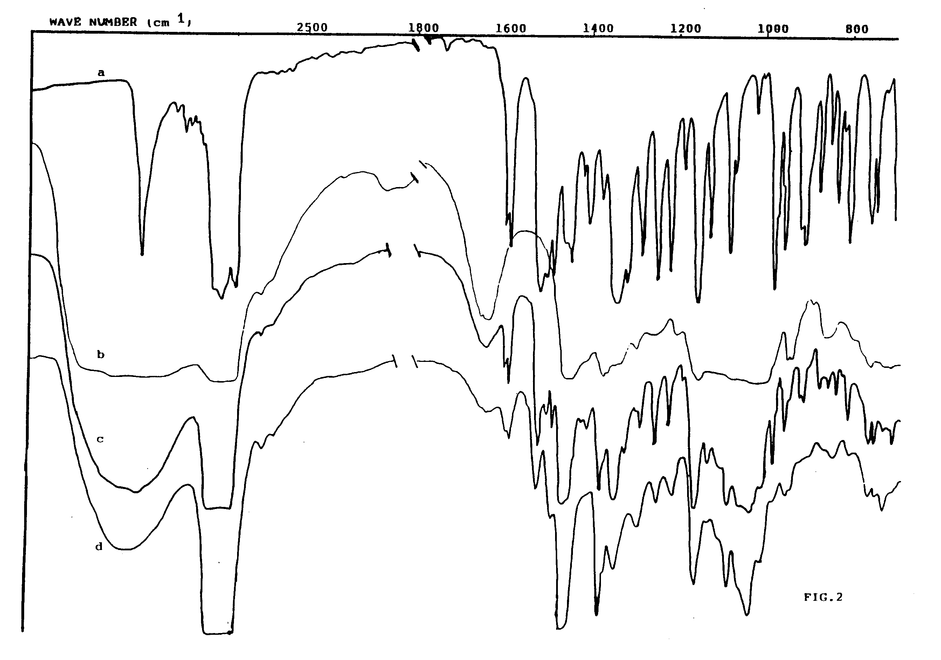

IR spectra of nimesulide (a), of β-cyclodextrin (b), of 1 : 1 (mol : mol) physical mixture (c) and of the inclusion compound (d) are shown in the figure 2. Comparing the spectrum of the inclusion compound with that of the physical mixture, there are evident modifications in the bands around 1520 cm$^{-1}$, due to the asymmetric stretching of nitro group, and especially in the region 1300 - 1200 cm$^{-1}$, giving proof of the significant differences existing between the inclusion compound and the physical mixture, according to the conclusions drawn from the differential scanning calorimetry results.

Bioavailability and pharmacokinetic

Plasma kinetics were carried out in dog after oral administration of equivalent dosages of nimesulide and inclusion compound nimesulide -β-cyclodextrin according to a crossover scheme.

Four male Beagle of body weight between 12.8 and 13.9 kg, fasted for at least 17 hours, were orally administered the following preparations:

A) nimesulide at the dose of 10 mg/kg

B) inclusion compound nimesulide -$\beta$-cyclodextrin (molar ratio 1 : 1) at the dose of 46.84 mg/Kg, corresponding to 10 mg/kg of nimesulide.

The preparations were administered in gelatine capsule after a "wash-out" period of 10 days.

The blood samples from dogs were collected in heparinized tubes after 1, 2, 3, 4, 5, 6, 8, 24, 28, 32, 48 and 52 hours from the administration of the two preparations. The relative plasma samples were obtained by centrifugation for 15 minutes at 3000 rpm.

The plasma levels of nimesulide were determined by HPLC with UV detection according to the following procedure: aliquots of plasma up to 2.5 ml were adjusted to 3.5 ml with distilled water and acidified with 1.0 ml of N hydrochloric acid and poured on to a glass column (18 mm i.d. 220 mm of lenght) containing 15 ml of Extralat® Merck. After 15 minutes nimesulide was eluted with two aliquotes of 7.5 ml of dichloromethane containing 5% of methanol. The eluate was evaporated to dryness under reduced pressure and the residue was taken up with 200 $\mu$l of 2.5 $\mu$g/ml solution of butilhydroxyanisole as internal standard. A suitable amount was therefore injected into a column by using the following chromatographic conditions:

- Column RPC$_8$, 5 $\mu$m, 250 x 3 mm i.d. Supelco;
- Mobile phase : acetonitrile + water containing 80 mg/l of EDTA in the ratio V/V 37.5 and 62.5 respectively;
- Flow rate : 1.0 ml/min.
- $\lambda$ of detection: 230 nm

The obtained results are reported in table 1.

The parameters of AUC. $C_{max}$ and $T_{max}$ were calculated by the plasma levels of nimesulide using a computer fitting system with TOP FIT package and the results were statistically evaluated by the ANOVA TWO WAYS procedure.

The results of the kinetic parameters are reported in the table 2 and are statistically highly significative.

From the above reported results it can be concluded that the inclusion compound nimesulide-$\beta$ -ciclodextrin increases significatively the bioavailability and the maximum plasma concentration ($C_{max}$) of nimesulide.

EP 0 534 995 B1

**TABLE I**    Plasma levels of Nimesulide  in the dog after oral administration of equivalent dosages
of Nimesulide (10mg/Kg.)Ⓐand nimesulide-$\beta$-cyclodextrin inclusion compound Ⓑ

| PREPARATION | Dogs No. | means ± S.E. | Nimesulide plasma levels   ( $\mu$g/ml.) hours after administration | | | | | | | | | | | |
|---|---|---|---|---|---|---|---|---|---|---|---|---|---|---|
| | | | 1 | 2 | 3 | 4 | 5 | 6 | 8 | 24 | 28 | 32 | 48 | 52 |
| A | 4 | $\overline{X}$ ± | 2.735 | 7.439 | 10.855 | 13.045 | 12.687 | 12.125 | 9.235 | 1.490 | 1.182 | 0.802 | 0.166 | 0.136 |
| | | $\overline{S.E.}$ | 0.820 | 0.579 | 0.929 | 2.071 | 2.580 | 2.519 | 1.426 | 0.572 | 0.461 | 0.335 | 0.112 | 0.104 |
| B | 4 | $\overline{X}$ ± | 11.040 | 22.130 | 25.277 | 26.060 | 23.817 | 22.890 | 19.447 | 2.937 | 1.480 | 0.992 | 0.156 | 0.111 |
| | | $\overline{S.E.}$ | 2.265 | 1.552 | 1.417 | 2.445 | 1.887 | 1.779 | 2.028 | 1.253 | 0.379 | 0.280 | 0.070 | 0.050 |

TABLE 2

| Kinetic parameters | | | | | |
|---|---|---|---|---|---|
| Preparation | dogs No. | Means ± S.E. | AUC (0→00) $\mu$g.h/ml | $C_{max}$ $\mu$g/ml | $T_{Cmax}$ h |
| A | 4 | X ± S.E. | 165.359 38.817 | 12.112 1.733 | 4.4 0.5 |
| B | 4 | X ± E.S. | 320.750 37.988 | 25.630 1.384 | 3.6 0.4 |

According to a further feature of the present invention there are provided pharmaceutical compositions comprising, as active ingredient, nimesulide complexed by inclusion into cyclodextrins in the above defined ratios, in association with one or more pharmaceutical carriers, excipients or diluents.

For the pharmaceutical administration nimesulide complexed by inclusion into cyclodextrin may be incorporated into conventional pharmaceutical compositions in either solid or liquid form. The compositions may be presented, for example, in a suitable form for oral, rectal, parenteral administration or for topic use. Preferred forms include, for example, capsules, tablets, coated tablets, granules, ampoules, suppositories, oral drops, syrup, emulsion, gel, ointment and sustained release compositions.

The inclusion compound nimesulide-cyclodextrin may be formulated, with the excipients or carrier conventionally used in the pharmaceutical compositions such as, for example, talc, arabic gum, lactose, gelatine, magnesium stearate, corn starch, aqueous or non-aqueous vehicles, polyvinylpirrolidone, semisynthetic glicerides of fatty acids, sorbitol, glycerol vaseline, hydroxyethylcellulose, propylene glycol, citric acid, sodium citrate, flavouring, sweeteners, binders and disgregating agents.

The compositions are advantageously formulated in dosage unit; each dosage unit being adapted to supply a single dose of the active ingredient. Each dosage unit may conveniently contain from 5 to 300 mg, preferably from 20 to 150 mg of the active ingredient.

The following non-limitative examples illustrate the pharmaceutical compositions according to the present invention:

Example 5

Tablets (wet granulation)

| | |
|---|---|
| - Nimesulide -$\beta$-cyclodextrin (1 : 1) (equivalent to 50 mg of nimesulide) | 240 mg |
| - hydroxypropylmethylcellulose | 70 mg |
| - microcrystalline cellulose | 150 mg |
| - colloidal silica | 5 mg |
| - magnesium stearate | 5 mg |
| | 470 mg |

| | |
|---|---|
| - Nimesulide -$\beta$-cyclodextrin (1 : 1) (equivalent to 100 mg of nimesulide) | 480 mg |
| - hydroxypropylmethylcellulose | 120 mg |
| - microcrystalline cellulose | 220 mg |
| - colloidal silica | 10 mg |
| - magnesium stearate | 10 mg |
| | 840 mg |

Method of preparation: the binder solution consisting of hydroxypropylmethylcellulose was used for the granulation of nimesulide and $\beta$-cyclodextrin, previously mixed with microcrystalline cellulose. After suitable drying the granulate so obtained was classified and mixed with colloidal silica and with magnesiun stearate. The composition was therefore pressed in order to obtain tablets containing 50 and 100 mg of nimesulide

7

Example 6

Tablets (direct compression)

| | |
|---|---|
| - Nimesulide -$\beta$-cyclodextrin (1 : 1) (equivalent to mg 50 of nimesulide) | 240 mg |
| - lactose | 40 mg |
| - calcium phosphate | 60 mg |
| - magnesium stearate | 2 mg |
| - colloidal silica | 3 mg |
| | 345 mg |

| | |
|---|---|
| - Nimesulide -$\beta$-cyclodextrin (1 : 1) (equivalent to 100 mg of nimesulide) | 480 mg |
| - lactose | 80 mg |
| - calcium phosphate | 120 mg |
| - magnesiun stearate | 4 mg |
| - colloidal silica | 6 mg |
| | 690 mg |

Method of preparation: nimesulide in the form of inclusion compound with $\beta$-cyclodextrin was diluted progressively with lactose and calciun phosphate. To the mixture so obtained magnesiun stearate and colloidal silice were added. After a suitable mixing the composition was pressed in order to obtain tablets containing each 50 and 100 mg of nimesulide respectively.

Example 7

Capsules

| | |
|---|---|
| - Nimesulide -$\beta$-cyclodextrin (1 : 1) (equivalent to 50 mg of nimesulide) | 240 mg |
| - hydroxypropylmethylcellulose | 80 mg |
| - magnesium stearate | 10 mg |
| | 330 mg |

| | |
|---|---|
| - Nimesulide -$\beta$-cyclodextrin (1 : 1) (equivalent to 100 mg of nimesulide) | 480 mg |
| - hydroxypropylmethylcellulose | 160 mg |
| - magnesium stearate | 15 mg |
| - colloidal silica | 5 mg |
| | 660 mg |

Method of preparation: nimesulide in the form of inclusion compound with $\beta$-cyclodextrin was suitably diluted with the excipients and filled into gelatine capsule. Each capsule contains 50 and 100 mg of nimesulide respectively.

Example 8

Granules

| - Nimesulide -β-cyclodextrin (1 : 1) (equivalent to 50 mg of nimesulide) | 240 mg |
|---|---|
| - sorbitol | 2710 mg |
| - aspartame | 15 mg |
| - raspberry flavour | 35 mg |
| | 3000 mg |

Method of preparation: nimesulide in the form of inclusion compound with β-cyclodextrin was progressively diluted with sorbitol. After having mixed aspartame and flavor agent were added. Therefore, the mixture in the form of powder so obtained was filled into sachets. Each sachet contains 50 mg of nimesulide.

Example 9

Monodose containers

| - Nimesulide -β-cyclodextrin (1 : 1) (equivalent to 50 mg of nimesulide) | 240 mg |
|---|---|
| - mannitol | 500 mg |
| - raspberry flavour | 40 mg |
| - sodium saccharinate | 5 mg |
| - phosphates | 5 mg |
| | 785 mg |
| - Deionized water | 19625 mg |

Method of Preparation: mannitol was dissolved in a pH 7.1 buffered solution and then nimesulide in the form of inclusion compound with β-cyclodextrin was added under slight stirring at room temperature. The raspberry flavour and colouring agent were then added. The solution was filtered and filled into vials and then freeze-dried. Each vial contains 50 mg of nimesulide.

Example 10

Suppositories

| - Nimesulide -β-cyclodextrin (1 : 1) (equivalent to 50 mg of nimesulide) | 240 mg |
|---|---|
| -semisynthetic glycerides of fatty acids | 1260 mg |
| | 1500 mg |

| - Nimesulide -β-cyclodextrin (1 : 1) (equivalent to 100 mg of nimesulide) | 480 mg |
|---|---|
| - semisynthetic glycerides of fatty acid | 1520 mg |
| | 2000 mg |

Method of preparation: the suppository mass was melted and nimesulide in the form of inclusion compound with β-cyclodextrin was added under continuous and constant stirring. Then, it was cooled at a proper temperature until the mass so obtained was poured into performed moulds for suppositories. After cooling suppositories containing 50 or 100 mg of nimesulide were obtained.

Example 11

Oral drops

| | |
|---|---|
| - Nimesulide -$\beta$-cyclodextrin (1 : 1) (equivalent to 50 mg of nimesulide) | 240 mg |
| - sodium saccharinate | 40 mg |
| - propylene glycol | 400 mg |
| - flavour | 5 mg |
| - demineralized water | 515 mg |
| | 1200 mg |

Method of preparation: sodium saccharinate was dissolved in water under slight stirring, then nimesulide in the form of inclusion compound with $\beta$-cyclodextrin was added. The resulting solution was diluted by dropping propylene glycol and lastly the flavour was added. The solution so obtained was distributed in 30 ml polyethylene bottle and dosed by a proper dropper in order to obtain 20 drops (1 ml) for a dosage of 50 mg of nimesulide.

Example 12

Syrup

| | |
|---|---|
| - Nimesulide -$\beta$-cyclodextrin (1 : 1) (equivalent to 50 mg of nimesulide) | 2400 mg |
| - hydroxyethylcellulose | 200 mg |
| - 70% sorbitol solution | 50000 mg |
| - glycerol | 12750 mg |
| - benzoic acid | 200 mg |
| - tartaric acid | 100 mg |
| - raspberry flavour | 330 mg |
| - deionized water | 50580 mg |
| | $\cong$ 100.0 mg |

Method of preparation: hydroxyethylcellulose was dispersed in the proper amount of the warm water until a solution was obtained. Then benzoic acid, tartaric acid, glycerol and then nimesulide in the form of inclusion compound with $\beta$-cyclodextrin were added under slight stirring. To the so obtained solution sorbitol solution was added, and lastly the flavour. Each spoon ($\cong$ 10 ml) contains 50 mg of nimesulide.

Example 13

Ointment

| | |
|---|---|
| - Nimesulide -$\beta$-cyclodextrin (1 : 1) (equivalent to 2,5 g. of nimesulide) | 11.7 g. |
| - vaseline-oil | 12.3 g. |
| - white vaseline | 76.0 g. |
| | 100.0 g. |

Method of preparation: nimesulide in the form of inclusion compound with $\beta$-cyclodextrin was dispersed in the vaseline oil at the temperature between 45-50 ° C. At this stage the white vaseline, previously heated at the same temperature, was added. The mixing stage was completed under vacuun by a turbo-mixer. Then the mass was cooled reducing also the stirring intensity. At the end the ointment was put into aluminium tubes (100 g/tube).

## Claims

1. Compounds obtained by complexation of nimesulide with an unsubstituted or substituted $\alpha$-, $\beta$- or $\gamma$-cyclodextrin, or a hydrate thereof in a molar ratio comprised between 1 : 0.5 and 1 : 15 of nimesulide and cyclodextrin respectively.

2. Compounds according to claim 1 wherein the molar ratio of nimesulide/cyclodextrin is 1 : 1.

3. Compounds according to claims 1 and 2 wherein the cyclodextrin is $\beta$-cyclodextrin.

4. Process for the preparation of the compounds according to claims 1 - 3, characterized in that an aqueous solution of cyclodextrin is interacted with an organic solution of nimesulide from which the inclusion product is isolated by filtration.

5. Process for the preparation of the compounds according to claims 1 - 3, characterized in that nimesulide and cyclodextrin are interacted at room temperature and under stirring in a water-ammonia solution.

6. Process according to claim 5, characterized in that the inclusion compound is isolated by freeze-drying of the solution.

7. Process according to claim 5, characterized in that the inclusion compound is isolated by atomization of the solution.

8. Pharmaceutical compositions having analgesic and antiinflammatory activity containing, as active ingredient, at least a compound according to claims 1 - 3.

9. Pharmaceutical compositions according to claim 8 containing from 20 to 150 mg of active ingredient for dosage unit.

10. Use of the compounds according to claims 1-3 for the manufacturing of a medicament for the treatment of inflammatory and pain states.

## Patentansprüche

1. Verbindungen, erhalten durch Komplexierung von Nimesulid mit einem unsubstituierten oder substituierten $\alpha$-, $\beta$- oder $\gamma$-Cyclodextrin oder einem Hydrat davon in einem Molverhältnis von Nimesulid zu Cyclodextrin im Bereich von 1 : 0,5 bis 1 : 15.

2. Verbindungen nach Anspruch 1, wobei das Molverhältnis von Nimesulid/Cyclodextrin 1 : 1 beträgt.

3. Verbindungen nach Anspruch 1 und 2, wobei das Cyclodextrin $\beta$-Cyclodextrin ist.

4. Verfahren zur Herstellung der Verbindungen der Ansprüche 1 bis 3, dadurch gekennzeichnet, daß man eine wäßrige Cyclodextrinlösung auf eine organische Nimesulidlösung einwirken läßt, aus der das Einschlußprodukt durch Filtration isoliert wird.

5. Verfahren zur Herstellung der Verbindungen der Ansprüche 1 bis 3, dadurch gekennzeichnet, daß man Nimesulid und Cyclodextrin bei Raumtemperatur und unter Rühren in einer Wasser-Ammoniak-Lösung aufeinander einwirken läßt.

6. Verfahren nach Anspruch 5, dadurch gekennzeichnet, daß die Einschlußverbindung durch Gefriertrocknen der Lösung isoliert wird.

7. Verfahren nach Anspruch 5, dadurch gekennzeichnet, daß die Einschlußverbindung durch Zerstäuben der Lösung isoliert wird.

**8.** Pharmazeutische Mittel mit analgetischer und antiinflammatorischer Aktivität, die als aktiven Bestandteil wenigstens eine Verbindung der Ansprüche 1 bis 3 enthält.

**9.** Pharmazeutische Mittel nach Anspruch 8, die 20 bis 150 mg des aktiven Bestandteils als Dosiseinheit enthalten.

**10.** Verwendung der Verbindungen der Ansprüche 1 bis 3 zur Herstellung eines Medikaments zur Behandlung von inflammatorischen und schmerzhaften Zuständen.

**Revendications**

**1.** Composés obtenus par complexation de nimésulide avec une $\alpha$-, $\beta$- ou $\gamma$-cyclodextrine non substituée ou substituée ou un hydrate de celle-ci, dans un rapport molaire compris entre 1/0,5 et 1/15 respectivement du nimésulide et de la cyclodextrine.

**2.** Composés selon la revendication 1, où le rapport molaire nimésulide/cyclodextrine est de 1/1.

**3.** Composés selon les revendications 1 et 2, où la cyclodextrine est la $\beta$-cyclodextrine.

**4.** Procédé pour la préparation des composés selon les revendications 1 à 3, caractérisé en ce que l'on fait réagir l'une sur l'autre une solution aqueuse de cyclodextrine et une solution organique de nimésulide dont on isole par filtration le produit d'inclusion.

**5.** Procédé pour la' préparation des composés selon les revendications 1 à 3, caractérisé en ce que l'on fait réagir l'un sur l'autre le nimésulide et la cyclodextrine à la température ordinaire et sous agitation dans une solution aqueuse d'ammoniac.

**6.** Procédé selon la revendication 5, caractérisé en ce que le composé d'inclusion est isolé par lyophilisation de la solution.

**7.** Procédé selon la revendication 5, caractérisé en ce que le composé d'inclusion est isolé par atomisation de la solution.

**8.** Compositions pharmaceutiques ayant une activité analgésique et anti-inflammatoire contenant, en tant qu'ingrédient actif, au moins un composé selon les revendications 1 à 3.

**9.** Compositions pharmaceutiques selon la revendication 8, contenant de 20 à 150 mg d'ingrédient actif par dose unitaire.

**10.** Utilisation des compositions selon les revendications 1 à 3 pour la fabrication d'un médicament pour le traitement des états inflammatoires et douloureux.

FIG.1

HEAT FLOW EXOTHERMAL

FIG.2